# EUROPEAN PATENT APPLICATION

(11) **EP 3 257 481 A1**
(43) Date of publication of application: **20.12.2017**
(21) Application number: 15881703.1
(22) Date of filing: 24.06.2015
(51) Int. Cl.: A61F 2/90, A61L 27/04

(54) **MULTILAYERED EXPANDABLE VASCULAR SCAFFOLD**

(30) Priority: 10.02.2015 CN 201520095013 U
(71) Applicant: Dongguan Dianfu Product Design Co., Ltd., Dongguan, Guangdong 523000 (CN)
(72) Inventor: LI, Yangde, Dongguan Guangdong 523000 (CN)
(74) Representative: Hryszkiewicz, Danuta
(86) International application number: PCT/CN2015/082153
(87) International publication number: WO 2016/127542

(57) **Abstract**

Disclosed is a multilayered expandable vascular scaffold, comprising at least two layers of magnesium alloy latticed scaffold walls (10) and a scaffold inner cavity (20). An expandable balloon (30) is arranged inside the scaffold inner cavity (20). During the expansion of the multilayered expandable vascular scaffold, the scaffold wall close to the outer layer exceeds its elongation rate and fractures due to the relatively large expansion. By providing the multilayered latticed scaffold wall, the vascular scaffold is prevented from breaking and losing the scaffolding function caused by outer layer fracture during expansion, and the safety is further improved.

## Description

The invention relates to the field of vascular stents, and more particularly to a multilayered expansible vascular stent.

In recent years, magnesium alloys have been widely used in clinical applications as cardiovascular stent materials. Based on the principle that magnesium can degrade gradually and disappear in the human body, magnesium alloys as metal implants can restore the defects in vivo. Moreover, magnesium is the biological element of life, with good biocompatibility, unique degradation and excellent comprehensive mechanical properties. Therefore, magnesium has a very attractive and promising medical application prospect.

However, magnesium and magnesium alloy have a poor corrosion resistance, the standard potential of pure magnesium is -2.37V, which is even lower in the physiological environment containing chloride ions; in simulated body fluid, the corrosion degradation rate of magnesium and magnesium alloys can reach 0.05-6 mm/year. In addition, magnesium alloy stents implanted in cardiovascular system tend to fracture or collapse under the impact of blood flow, thus losing support function in vascular remodeling, even causing death, which largely limits the application of the magnesium alloy stent in the coronary stent.

In view of the above-described problems, it is one objective of the invention to provide a multilayered expansible vascular stent. In the expansion process of the vascular stent, the support walls in the outer layer have a relatively large expansion extent which exceeds the elongation percentage thereof, thus tend to break or damage. Through disposing multilayered magnesium alloy latticed vascular stent, the loss of support function of the stent resulting from the breakage of the outer layer support walls in the expansion process of the vascular stent is safeguarded, thus improving the security of the vascular stent, which is favorable to popularizing and using the vascular stent.

To achieve the above objective, in accordance with one embodiment of the invention, there is provided a multilayered expansible vascular stent comprising at least two layers of magnesium alloy latticed support walls, a cavity, and an expansible balloon, the expansible balloon being disposed in the cavity.

In a class of this embodiment, the latticed support walls are between 0.05 and 0.2 mm in thickness.

In a class of this embodiment, surfaces of the latticed support walls are coated with a drug-loading layer. The drug-loading layer comprises a polymer and an active component. The polymer is an ethylene-vinyl alcohol copolymer, ethylene-vinyl acetate copolymer or styrene-isobutylene block copolymer. The active component is antiplatelet drug.

Advantages of the multilayered expansible vascular stent in accordance with embodiments of the invention are summarized as follows. The multilayered expansible vascular stent comprises at least two layers of latticed support walls, when the stent expands, the latticed support walls in the outer layer have a relatively large expansion extent which exceeds the elongation percentage thereof, thus tend to break or damage, while the latticed support walls in the inner layer have a relatively small expansion extent, and do not break and damage, thus ensuring the integrity of the vascular stent, improving the security of the vascular stent, which is favorable to popularizing and using the vascular stent.
FIG. 1 is a schematic diagram of a multilayered expansible vascular stent in accordance with one embodiment of the invention;
FIG. 2 is a schematic diagram of a multilayered expansible vascular stent after being expanded in FIG. 1;
FIG. 3 is a schematic diagram of a multilayered expansible vascular stent in accordance with another embodiment of the invention; and
FIG. 4 is a schematic diagram of a multilayered expansible vascular stent after being expanded in FIG. 3.

For further illustrating the invention, experiments detailing a multilayered expansible vascular stent are described hereinbelow combined with the drawings. It should be noted that the following examples are intended to describe and not to limit the invention.

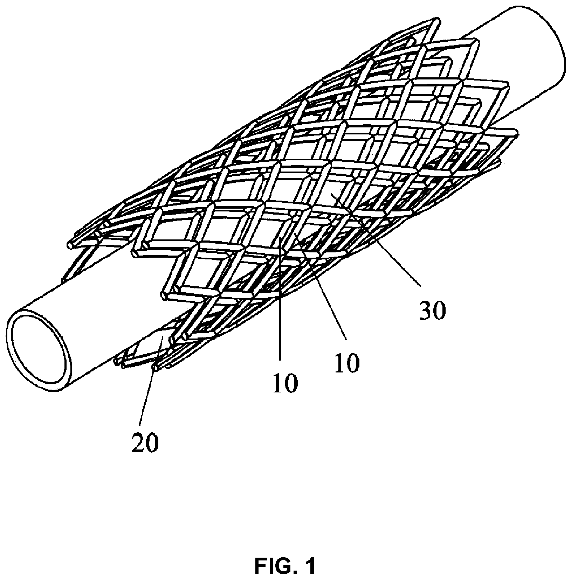

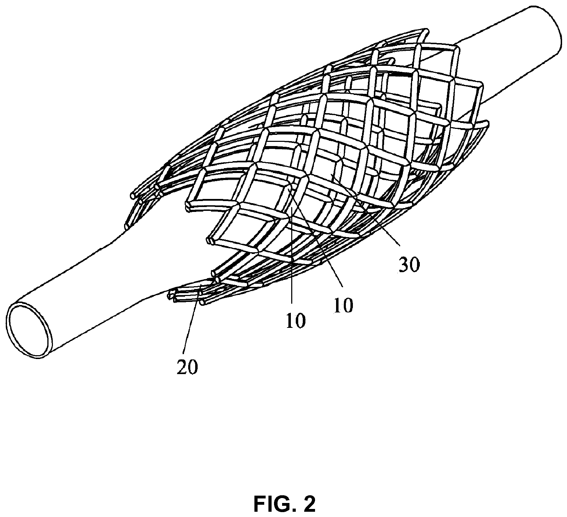

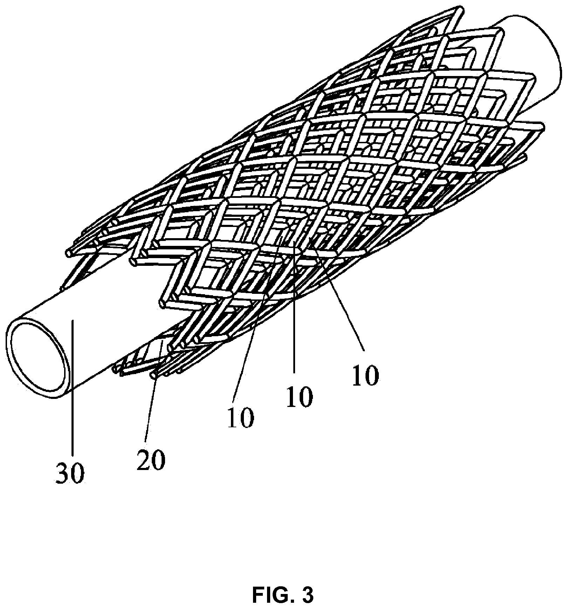

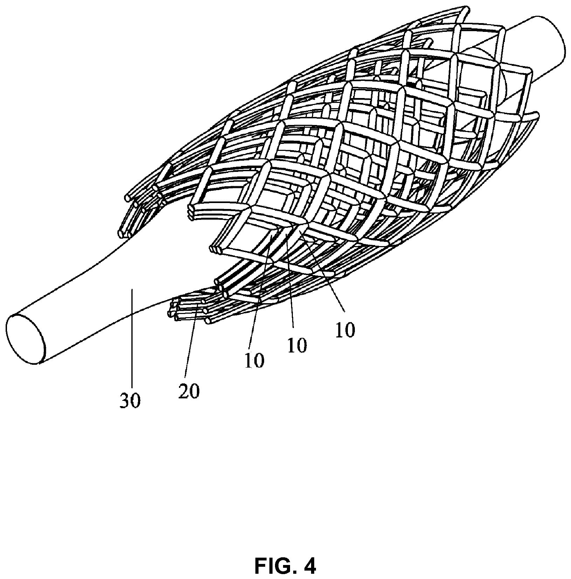

As shown in FIGS. 1, 2, 3, and 4, a multilayered expansible vascular stent comprises at least two layers of latticed support walls 10 and a cavity 20. An expansible balloon 30 is disposed in the cavity 20. The vascular vent is able to expand under the expansion of the balloon 30. The latticed support walls 10 in the outer layer have a relatively large expansion extent which exceeds the elongation percentage thereof, thus tend to break or damage, while the latticed support walls 10 in the inner layer have a relatively small expansion extent, and do not break and damage, thus ensuring the integrity of the vascular stent, and ensuring the vascular stent exhibits its role.

The latticed support walls are made of magnesium alloy material, which is degradable material and is degraded gradually with the accumulation of service time. Thus, according to the requirement for service life, reasonable quantity of latticed support walls is preset. In general, there are at least two or three layers of latticed support walls. The latticed support walls in each layer are between 0.05 and 0.2 mm in thickness.

Preferably, surfaces of the latticed support walls are coated with a drug-loading layer. The drug-loading layer comprises a polymer and an active component. The polymer is an ethylene-vinyl alcohol copolymer, ethylene-vinyl acetate copolymer or styrene-isobutylene block copolymer. The active component is antiplatelet drug. The stent is arranged in the blood vessel to prevent the restenosis of the blood vessel thus achieving the purpose of dredging the blood vessel. So, it is necessary to coat antiplatelet drug on the blood vessel, for example, cilostazol, which not only has the function of antiplatelet, but also has the function of expanding blood vessel, inhibits the growth of vascular smooth muscle, and inhibits thrombosis, including vascular wall and endothelial cell.

Unless otherwise indicated, the numerical ranges involved in the invention include the end values. While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. A multilayered expansible vascular stent, **characterized by,** comprising:
at least two layers of magnesium alloy latticed support walls;
a cavity; and
an expansible balloon which is disposed in the cavity.

2. The stent of claim 1, **characterized in that,** the latticed support walls are between 0.05 and 0.2 mm in thickness.

3. The stent of claim 1, **characterized in that,** surfaces of the latticed support walls are coated with a drug-loading layer; the drug-loading layer comprises a polymer and an active component; the polymer is an ethylene-vinyl alcohol copolymer, ethylene-vinyl acetate copolymer or styrene-isobutylene block copolymer; and the active component is antiplatelet drug.
